# EUROPEAN PATENT APPLICATION

(11) **EP 4 438 001 A1**
(43) Date of publication of application: **02.10.2024**
(21) Application number: 24166383.0
(22) Date of filing: 26.03.2024
(51) Int. Cl.: A61C 3/06

(54) **DENTAL POLISHING INSTRUMENT**

(30) Priority: 29.03.2023 JP 2023052704; 20.12.2023 JP 2023214952
(71) Applicant: Kabushiki Kaisha Shofu, Kyoto-shi Kyoto 605-0983 (JP)
(72) Inventor: TAKEHISA, Junya, Kyoto, 605-0983 (JP); TORITA, Yasuhiro, Kyoto, 605-0983 (JP); YUMIYAMA, Naoki, Kyoto, 605-0983 (JP); ISHIDA, Sayaka, Kyoto, 605-0983 (JP)
(74) Representative: Eisenführ Speiser

(57) **Abstract**

A dental polishing instrument capable of polishing a surface of a dental restoration or a tooth effectively and continuously, without the need to use abrasive agent such as a dental polishing paste, in combination with the dental polishing instrument will be provided. A dental polishing instrument according to the present invention includes: a porous base member (A); and a polishing composition that contains abrasive grains (B) and a water-soluble binder (C), and that is held by the porous base member.

## Description

### BACKGROUND

### 1. Technical Field

The present invention relates to a dental polishing instrument for polishing dental restorations such as those made of a composite resin, a ceramic, or a metal, and/or for cleaning and/or removing deposits on the tooth surface.

### 2. Description of the Related Art

In general dental treatment practices, when a tooth has a disease such as caries, the dental tissue is removed from the carious part, and the removed part is restored using a dental restoration made of various types of material such as a composite resin, a ceramic, or a metal.

When such a dental restoration has a rough texture or scratch on the surface, the dental restoration may become a cause of stains or plaque deposition. Therefore, before filling or attaching a dental restoration, the dental restoration after the shape correction and occlusal adjustment needs to be polished so that the dental restoration has a smooth surface. If the surface of the dental restoration is not smooth, the restoration can neither achieve naturalness equivalent to that of natural teeth, nor present a highly aesthetic appearance. Furthermore, if the texture of the surface of the dental restoration is not natural, the patient may feel something strange on the tongue and feel uncomfortable, and proper movements of oral mucosa and tongue may also be obstructed. For these reasons, it is very important to polish the surface of a dental restoration smoothly, and various dental polishing instruments have been used to achieve this goal.

In the step of final finish for achieving a smooth surface, it has been considered effective to use an abrasive such as a dental polishing paste that contains fine abrasive grains in the final-finish polishing. Generally, such abrasive agents are mainly used in free abrasive grain polishing, in which an appropriate amount of the abrasive agent is applied to an instrument such as a dental felt or a dental brush, and the instrument is rotated to polish an object to be polished, by bringing the abrasive agent into contact with the object.

In addition, at the dental office, tooth surface cleaning referred to as Professional Mechanical Tooth Cleaning (PMTC) is carried out by experts (operators) such as dentists and dental hygienists, for the purpose of removing substances such as a stain, dental plaque, and calculus having deposited on the tooth surface. In order to prevent dental caries and periodontal diseases, it is effective to remove substances deposited on the tooth surface, and removing of such substances contributes greatly in maintaining the health of the oral cavity. Furthermore, polishing also is important for aesthetic purposes, because the original beautiful tooth surface can be restored by removing the deposits on the tooth surface.

Free abrasive grain polishing is also used in cleaning the tooth surface for the purpose of removing deposits from the tooth surface, by applying an abrasive agent such as special dental polishing paste to a rotary instrument such as a dental brush or a dental prosthetic tip.

However, in these free abrasive grain polishing, it is necessary to apply an abrasive agent such as a dental polishing paste to the instrument such as a dental felt, which is used with the abrasive agent. In addition, part of the abrasive agent applied to the surface of such an instrument may scatter inside the oral cavity, due to the centrifugal force of the rotation, and the abrasive agent required for polishing may fall short. Reapplication of the abrasive may therefore become necessary. As described above, not only the operator needs to make some cumbersome movements, but also an extended time is required for polishing. Therefore, the operator has needed to bear a large burden. In order to shorten such a polishing time, there is a demand for a dental polishing instrument capable of performing effective and continuous polishing without the need for additional use of the abrasive material agent.

JP 2004-73543 A (Patent Document 1) discloses a technology for a dental cleaning/polishing profile tip that makes the use of a separate abrasive agent unnecessary, by using a hydrophilic sponge that contains abrasive grains and other various components. According to JP 2004-73543 A, by wetting the hydrophilic sponge with water before polishing, the components such as the abrasive grains contained in the sponge are dissolved and released into the water, as the hydrophilic sponge is frictionally rotated and pressed against teeth, so that the teeth can be polished by the free abrasive grains.

However, with the mechanism disclosed in JP 2004-73543 A, because the abrasive grains are merely released and held by the water contained in the hydrophilic sponge during the polishing, with no abrasive grains themselves being released gradually from the internal of the mechanism over some extended time, such a mechanism is incapable of sustaining the polishing effect sufficiently. Furthermore, because no particular optimization is made as to the hardness of the sponge that is a porous base member, it is unclear whether the sponge is capable of polishing effectively by deforming in a manner following a shape such as the that of the occlusal surface of a molar, as appropriate.

### SUMMARY

An object of the present invention is to provide a dental polishing instrument capable of polishing a surface of a dental restoration or a tooth effectively and/or continuously, without the need to use abrasive agent such as a dental polishing paste, in combination with the dental polishing instrument.

As a result of intensive studies for overcoming the issues described above, the inventors of the present patent application have come up with the present invention. That is, a dental polishing instrument according to the present invention includes:
a porous base member (A); and
a polishing composition that contains abrasive grains (B) and a water-soluble binder (C), and that is held by the porous base member.

With the dental polishing instrument according to the present invention, it is possible to polish dental restorations and/or teeth effectively and/or continuously, without applying any abrasive such as a dental polishing paste.

### BRIEF DESCRIPTION OF DRAWINGS

Fig. 1(a) is a schematic view illustrating a shape of a dental polishing instrument according to a first embodiment, and Fig. 1(b) is an enlarged cross-sectional view illustrating a cross-sectional structure of a part of Fig. 1(a);
Fig. 2 is a schematic perspective view illustrating a method for measuring 40% hardness of a porous base member, in accordance with JIS K 6400-2 hardness A method;
Fig. 3A is a schematic perspective view of a dental polishing instrument including a shaft, according to a second embodiment;
Fig. 3B is a schematic perspective view of a dental polishing instrument according to a modification of the second embodiment, with the shaft of the dental polishing instrument separated;
Fig. 4A is a schematic front view of the dental polishing instrument illustrated in Fig. 3A;
Fig. 4B is a schematic front view of the dental polishing instrument illustrated in Fig. 3B;
Fig. 5 is a schematic perspective view illustrating a shape of a test piece used for evaluating polishing performance;
Fig. 6A is a schematic front view illustrating a bullet-shaped porous base member;
Fig. 6B is a schematic front view illustrating a spindle-shaped porous base member;
Fig. 6C is a schematic front view illustrating a cup-shaped dental porous base member;
Fig. 7 is Table 1 indicating the types, the concentrations, and the solvents of the water-soluble binder used in the solution of the water-soluble binder (C) used in examples and comparative examples;
Fig. 8 is Table 2 indicating the compositions and mixed ratios of polishing composition slurries, and the materials of the porous base member (A); and
Fig. 9 is Table 3 indicating the 40% hardness of the porous base members (A), the abrasive grains (B), the water-soluble binder (C), and solutions thereof used in Example 1 to 11 and Comparative Example 1.

### DETAILED DESCRIPTION

Detailed descriptions of the present invention will be provided below. Note that the present invention is not limited to the particular example described below.

A dental polishing instrument according to a first aspect includes:
a porous base member (A); and
a polishing composition that contains abrasive grains (B) and a water-soluble binder (C), and that is held by the porous base member.

Further, as a dental polishing instrument of a second aspect, in the first aspect, the abrasive grains may be released from the porous base member when the polishing composition is brought into contact with water or a liquid containing alcohol.

Further, as a dental polishing instrument of a third aspect, in the first aspect, the water-soluble binder (C) may be at least one selected from the group consisting of polyvinylalcohol, polyvinylpyrrolidone, polyacrylate, polyethylene glycol, alginate, pullulan, carrageenan, hydroxyethyl cellulose, and methyl cellulose.

Further, as a dental polishing instrument of a fourth aspect, in the first aspect, a weight ratio of the polishing composition may be set to 0.4 or higher, representing a dry weight of the porous base member (A) alone as 1.

Further, as a dental polishing instrument of a fifth aspect, in the first aspect, 40% hardness of the porous base member (A), resultant of applying a compressive load to the porous base member (A) alone in accordance with JIS K 6400-2 (corresponding to ISO 2439:2008, ISO 3386-1:1986, and ISO 3386-2:1997) hardness A method, may be within a range of 3 N to 18 N.

Further, as a dental polishing instrument of a sixth aspect, in the first aspect, the porous base member may have any one of a bullet-like shape, a spindle shape, a cylindrical shape, a conical shape, a paraboloid shape, and a cup shape.

A polishing method according to a seventh aspect, includes:
bringing the dental polishing instrument according to the first aspect into contact with a dental restoration or a tooth surface; and
polishing the dental restoration or the tooth surface while supplying water or a liquid containing alcohol to the dental abrasive.

Dental polishing instruments according to some embodiments will now be explained with reference to the appended drawings. In the drawings, substantially the same members are denoted by the same reference numerals.

### (First Embodiment)

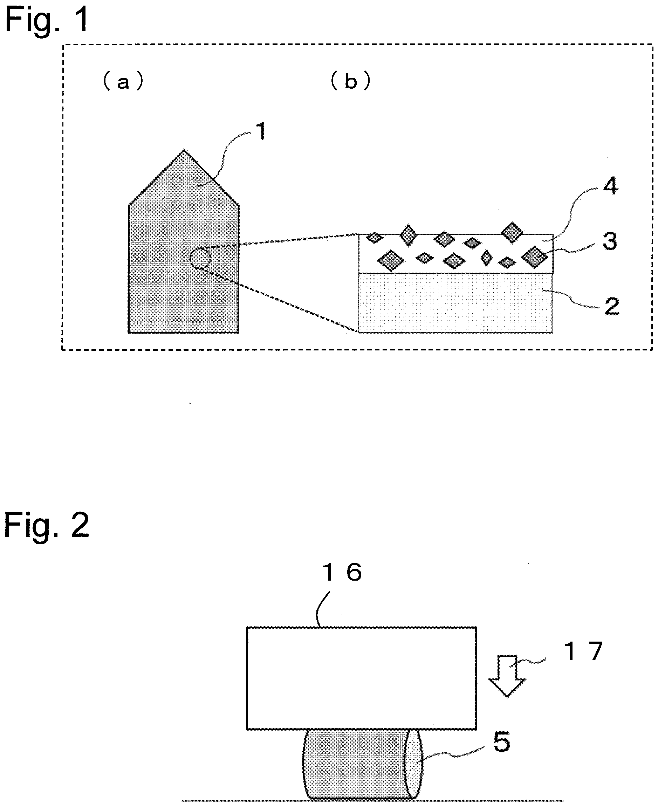

Fig. 1(a) is a schematic view illustrating a shape of a dental polishing instrument 1 according to a first embodiment, and Fig. 1(b) is an enlarged cross-sectional view illustrating a cross-sectional structure of a part of the dental polishing instrument 1 illustrated in Fig. 1(a).

The dental polishing instrument 1 according to the first embodiment includes a porous base member 2 (A) and a polishing composition that contains abrasive grains 3 (B) and a water-soluble binder 4 (C), and that is held by the porous base member 2.

With the dental polishing instrument 1, the water-soluble binder 4 dissolves into the water or the liquid containing alcohol by being brought into contact therewith, thereby allowing the abrasive grains 3 to be gradually released from the porous base member 2, so that polishing can be continued.

Members included in the dental polishing instrument will be described below.

### <Porous base member>

The material of the porous base member (A) is not limited to a particular material, but preferably, a material formed of fibers, such as nylon, polyethylene terephthalate, polyurethane, aramid, wool, and nonwoven fabric, is used. Examples of other materials include porous bodies made of plastics such as polyethylene, polypropylene, polystyrene, ethylene-vinyl acetate copolymer, and polymethyl methacrylate.

Fig. 2 is a schematic perspective view illustrating a method for measuring 40% hardness of a porous base member, in accordance with JIS K 6400-2 (corresponding to ISO 2439:2008, ISO 3386-1:1986, and ISO 3386-2:1997) hardness A method.

In order to polish a complicated shape such as the occlusal surface of a molar, effectively, moderate flexibility is required. Therefore, the porous base member (A) preferably has 40% hardness within the range of 3 N to 18 N, and particularly preferably within the range of 3 N to 12 N, when a compressive load 17 is applied to the porous base member (A) alone. The 40% hardness represents a load resultant of applying a compressive load 17 to a cylindrical test piece 5 having Φ 5 to 6 × a height of 6 mm to 7 mm, at an application speed of 100 mm/min using a load cell 16 or the like, to compress the test piece to 40% of its diameter, and allowing the test piece 5 to sit for 30 seconds in this condition, as illustrated in Fig. 2. The load 17 of the 40% hardness was used as an index of hardness. As to the test conditions such as the speed at which the load 17 is applied, the amount by which the porous base material is compressed, and the time for which the porous base material is left sitting after the compression, those stipulated in JIS K 6400-2 hardness A method was used. With 40% hardness less than 3 N, the porous base material is too soft to withstand the rotation during polishing. With the 40% hardness exceeding 18 N, because the porous base material is too hard to deform appropriately along a complicated shape such as the occlusal surface of a molar, at the time of polishing, only a small area can be polished at a time, so that the polishing efficiency deteriorates.

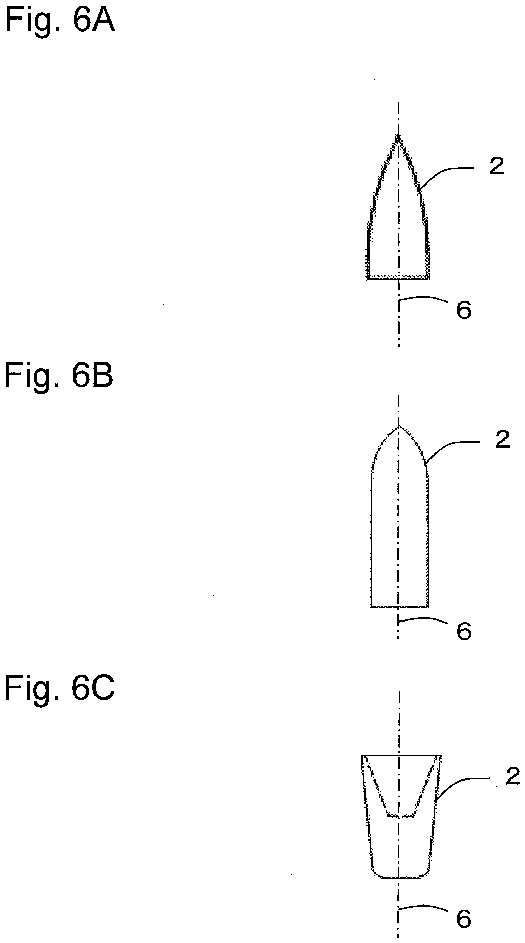

The shape of the porous base member (A) is not particularly specified, but a disk shape, a cup shape, or the like may be used preferably for the front teeth or the adjacent surface. For applications for the polishing of a complicated shape such as the occlusal surface of a molar, a bullet-like shape (Fig. 6A), a conical shape, a cylindrical shape, a spindle shape (Fig. 6B), a cup shape (Fig. 6C), or the like may be generally used. Furthermore, the porous base member delineating the outer shape of the dental polishing instrument may be rotationally symmetric with respect to the rotational axis 6, because dental polishing instruments are generally used in rotation. Furthermore, preferably, the porous base member has a maximum diameter of 2 mm to 10 mm, for example, with respect to the rotational axis 6, and a height that is a length of 5 mm to 30 mm, for example, along the direction of the rotational axis 6. In addition, the shape delineated by the surface of the porous base member (A) is not limited to any particular shape, but may have a three-dimensional shape, such a shape with slits, dimples, or protrusions, for the purpose of better holding the polishing composition.

### <Polishing composition>

As illustrated in Fig. 1(b), the polishing composition contains abrasive grains 3 and a water-soluble binder 4. The polishing composition may be held in the porous base member 2 (A) in any way. Fig. 1(b) illustrates the polishing composition being held on the inner surfaces of the pores in the porous base member 2, but the present invention is not limited thereto. The polishing composition may also be uniformly held on the surface of the porous base member 2 and the inner surfaces of the pores of the porous base member 2, as well as the internal of the porous base member itself. For example, the porous base member 2 (A) is immersed in a slurry of the polishing composition in which the abrasive grains 3 and the water-soluble binder 4 are dissolved and dispersed in water or a solvent containing alcohol, so as to impregnate the surface, the pore surfaces, and the internal of the porous base member 2 with the polishing composition. The solvent may then be removed by allowing to volatilize, so that the polishing composition is held in the porous base member. Examples of other methods include a method in which the slurry of the polishing composition is injected directly into the pores of the porous base member (A) or the internal of the porous base member itself using a syringe or the like, or the slurry is spray-coated and allowed to permeate through the surface into the pores or the internal of the porous base member itself, and then the solvent is removed by letting volatilize, in the same manner.

### <Abrasive grains (B)>

The type of the abrasive grains (B) is selected depending on factors such as the properties of the material of the object to be polished, and the purpose of final-finish polishing or deposit removal. Although the type is not limited to any particular type, for the final-finish polishing of a dental restoration, for example, one or more selected from the group of aluminum oxide, silicon carbide, diamond, boron nitride, and cerium oxide are preferably used. For the cleaning of tooth surface, as another example, one or more selected from the group of the following are preferably used, the group being a group of silica-based abrasive grains such as silicic anhydride, precipitated silica, silica gel, aluminosilicate, and zirconosilicate; a calcium phosphate-based compound such as second calcium phosphate dihydrate or anhydride, first calcium phosphate, third calcium phosphate, fourth calcium phosphate, and hydroxyapatite; calcium pyrophosphate; calcium carbonate; diatomaceous earth; aluminum hydroxide; and titanium oxide.

The abrasive grains (B) preferably have an average particle size of 30 µm or less, and particularly preferably an average particle size of 15 µm or less for the purpose of final-finish polishing, which is the step of final polishing of a dental restoration, or for the purpose of cleaning the tooth surface by removing deposits. When the average particle diameter is larger than 30 µm, a smooth surface cannot be achieved, disadvantageously, because the particle diameter is too large that new scratch is made. The average particle diameter herein is a median diameter (d50), and can be determined using a general granularity distribution meter, for example. There are granularity distribution meters using various measurement principles, and examples thereof include a granularity distribution meter using an electric resistance. The shape of the particles of the abrasive grains (B) is not limited to a particular shape.

The abrasive grains may be attached to a water-soluble binder, for example. Alternatively, the abrasive grains may be dispersed in a matrix that is a water-soluble binder. Furthermore, the abrasive grains may be dispersed uniformly across the water-soluble binder from the outer surface toward the inner side, in the depth direction. With this, when the water-soluble binder dissolves in water or a liquid containing alcohol, the abrasive grains are allowed to be released continuously and gradually.

### <Water-soluble binder>

The water-soluble binder (C) has two roles one of which is to hold or fix the abrasive grains (B) inside the porous base member (A), and the other one of which is to gradually become released with the abrasive grains (B) to the outside of the porous base member (A) by dissolving to water or a liquid containing alcohol, upon coming into contact therewith. In order to meet both of these requirements, the water-soluble binder preferably remains solid at 40 °C or lower, and exhibits water solubility. If the water-soluble binder (C) is semi-solid or liquid at 40 °C or lower, the force for holding or fixing the abrasive grains (B) inside the porous base member (A) falls short, and the polishing composition may come off easily or become sticky, so that the storage property deteriorates. In addition, if the binder (C) is not water-soluble, the binder (C) would not dissolve by coming into contact with water which is injected during polishing or saliva, and become incapable of releasing the abrasive grains (B) gradually to the outside of the porous base member (A).

The type of water-soluble binder (C) is not particularly specified as long as the requirements described above are met, but particularly preferable examples are polyvinylalcohol, polyvinylpyrrolidone, polyacrylate, polyethylene glycol, alginate, pullulan, carrageenan, hydroxyethyl cellulose, and methyl cellulose.

Although the water-soluble binder (C) is preferably solid at 40 °C or lower, it is also preferable for the water-soluble binder (C) to be handled as a solution when the porous base member (A) is impregnated therewith, together with the abrasive grains (B). The solvent used at that time is not particularly specified as long as the water-soluble binder (C) can dissolve thereinto. For example, the solvent is only required to be water or a liquid containing alcohol. Specifically, the solvent is only required to be water or at least one selected from a group of general alcohol-based solvents including ethanol, 1-propanol, 2-propanol, 1-butanol, 1-methoxy-2-propanol, ethylene glycol, propylene glycol, diethylene glycol monoethyl ether, and diethylene glycol.

The concentration of the water-soluble binder (C) with respect to the solution is not particularly specified, but is preferably 1 wt% to 20 wt%, for example. If the concentration of the water-soluble binder (C) with respect to the solution is lower than 1 wt%, the viscosity of the slurry that is a mixture of the binder and the abrasive grains (B) drops, so that the abrasive grains (B) precipitate and it becomes difficult to impregnate the porous base material (A) with the abrasive grains (B), stably. In addition, if the concentration is higher than 20 wt%, the slurry becomes highly viscous, and it becomes difficult to impregnate the porous base member (A) with the abrasive grains (B) and the water-soluble binder (C), so that the amount of the polishing composition, by which the base material is impregnated and contributing to polishing, becomes insufficient.

### <Others>

As the polishing composition with which the porous base member (A) is impregnated, any known components other than the water-soluble binder (C) and the abrasive grains (B) may be added as necessary, as long as the effects of the present invention are not inhibited thereby. For example, a thickener, a surfactant, a dispersant, a coloring agent, a sweetener, an antiseptic, a fragrance, or a medicinal ingredient may be added to the polishing composition. The way in which these components are added is not particularly limited, but preferably, such a component is dissolved to the solution of the water-soluble binder (C), or added at the same timing as the abrasive grains (B).

### <Method for manufacturing dental polishing instrument>

Although, in the present invention, a method for fabricating the dental polishing instrument is not limited to any particular method, as long as it is possible to achieve a dental polishing instrument including a porous base member (A) that is impregnated with a polishing composition containing the water-soluble binder (C) and the abrasive grains (B) and that has the polishing composition fixed thereto, the dental polishing instrument may be manufactured in the following manner, as one example.
(1) The slurry of the polishing composition is obtained by uniformly mixing the abrasive grains (B) into the solution of the water-soluble binder (C).
(2) The porous base member (A) is then immersed in the slurry of the polishing composition, to impregnate the porous base member with the slurry of the polishing composition.
(3) The solvent is then allowed to volatilize and removed using a hot air dryer, to obtain a dental polishing instrument in which a polishing composition containing abrasive grains and a water-soluble binder is held in a porous base member.

Representing the weight of the porous base member (A) alone during the drying step as 1, the weight ratio of the polishing composition content including the water-soluble binder (C) and the abrasive grains (B) is preferably 0.4 or higher, particularly preferably 1.1 or higher, with respect to the porous base member (A).

The weight ratio of the polishing composition content, representing the weight of the porous base member (A) alone during the drying process as 1, is calculated by the following formula. Weight Ratio of Polishing Composition Content= (Weight of Dental Polishing Instrument - Weight of porous base member (A) alone)/Weight of porous base member (A) Alone

### (Second Embodiment)

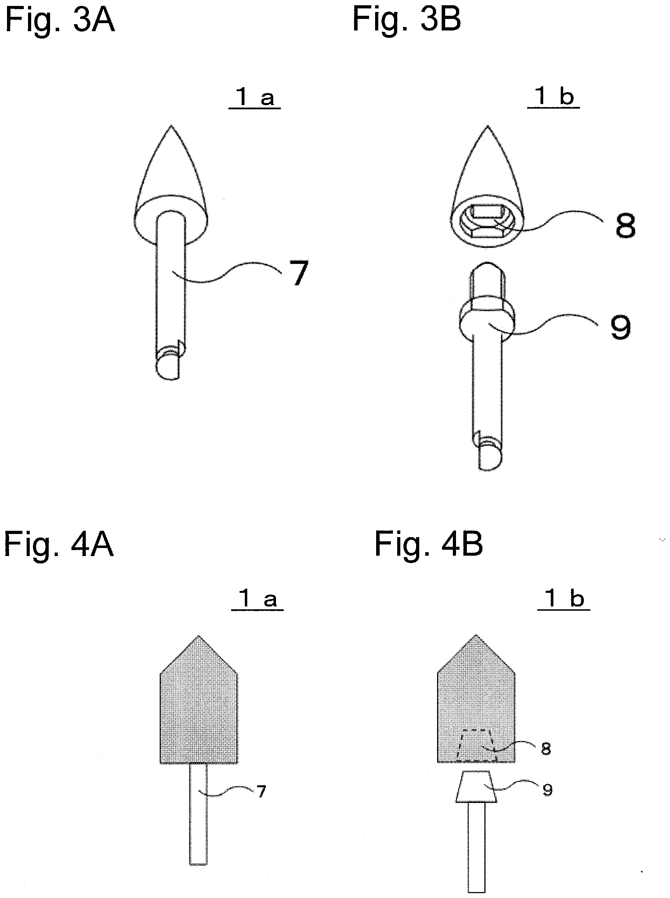

Fig. 3A is a schematic perspective view of a dental polishing instrument 1a including a shaft 7 according to a second embodiment, and Fig. 3B is a schematic perspective view of a dental polishing instrument 1b according to a modification of the second embodiment, with the shaft 9 of the dental polishing instrument 1b separated. Fig. 4A is a schematic front view of the dental polishing instrument 1a illustrated in Fig. 3A, and Fig. 4B is a schematic front view of the dental polishing instrument 1b illustrated in Fig. 3B.

The dental polishing instrument 1a according to the second embodiment is integrated with the shaft 7 for the attachment to the porous base member and a dental handpiece (not illustrated). Alternatively, the dental polishing instrument 1b according to the modification includes a shaft attachment 8 to and from which the shaft 9 can be attached and detached. The way in which the shaft 7 is fixed to the dental polishing instrument 1a according to the second embodiment is not particularly specified, but one example includes inserting the shaft 7 into the bottom surface of the porous base member (A), and fixing the shaft 7 to the dental polishing instrument 1a by crimping or boding with adhesive agent, in advance. In addition, to achieve a structure of the shaft 9 that is detachable, as in the dental polishing instrument 1b according to the modification, the shaft attachment 8 having a hole with a similar shape to the shape of the shaft 9 to be inserted is provided in the porous base member (A) so that the shaft 9 can be attached or detached thereto or therefrom, as appropriate, at the time of use.

### [Examples]

One example of the embodiment of the present invention will now be explained with using some examples. The examples described below are, however, not intended to limit the scope of the present invention in any way.

### <Fabrication of dental polishing instrument>

The dental polishing instruments according to examples and comparative examples were fabricated in the following manner.

(1-1) First, the solution of the water-soluble binder (C) was prepared by dissolving the water-soluble binder (C) into a solvent.

(1-2) The slurry of the polishing composition was then prepared by uniformly mixing the abrasive grains (B) into the solution of the water-soluble binder (C).

(2) A porous base member (A) having a shaft inserted into the bottom surface and fixed thereto in advance was then immersed in the slurry of the polishing composition for 10 seconds, to impregnate the porous base member with the slurry of the polishing composition.

(3) The polishing composition was then dried using a hot air dryer until the solvent volatilized, to achieve a dental polishing instrument impregnated with and having the polishing composition fixed thereto.

### <Examples and Comparative Examples>

In Example 1, aluminum oxide that is the abrasive grains (B) was uniformly mixed, by 150 parts by weight, to a solution 1 of polyvinylpyrrolidone that is the water-soluble binder (C) at 100 parts by weight (at a concentration of 10 wt% using 1-methoxy-2-propanol as a solvent), to obtain a slurry of a polishing composition.

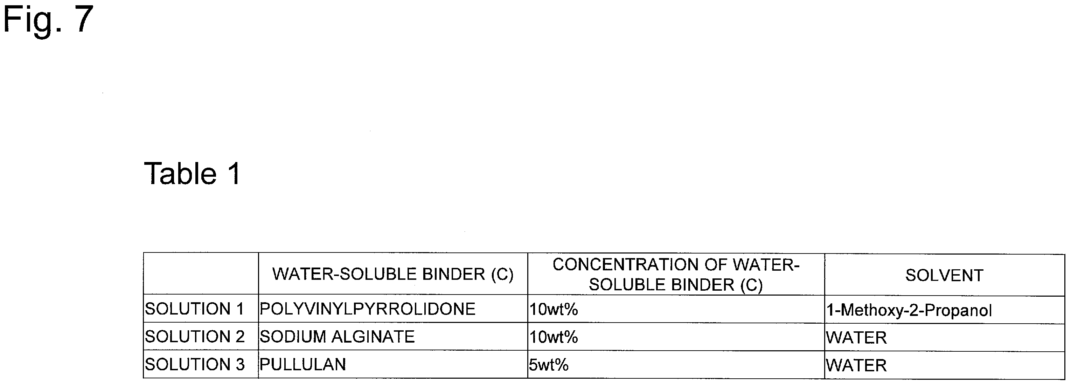

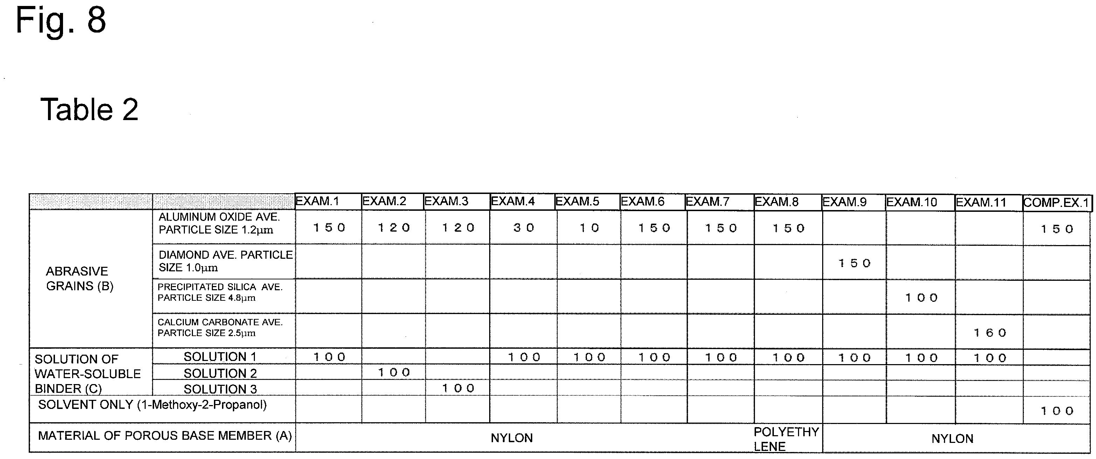

A bullet-shaped porous base member (A), made of nylon having 40% hardness of 12 N, was then impregnated with the obtained slurry of the polishing composition, and dried at 130 °C to achieve a dental polishing instrument. The solutions of the water-soluble binder (C) used in the other examples and comparative examples were also prepared following the same steps but with the compositions listed in Table 1 in Fig. 7. The compositions of the polishing composition slurry and the ratio of the components added thereto, and the materials used as the porous base member (A) are listed in Table 2 in Fig. 8.

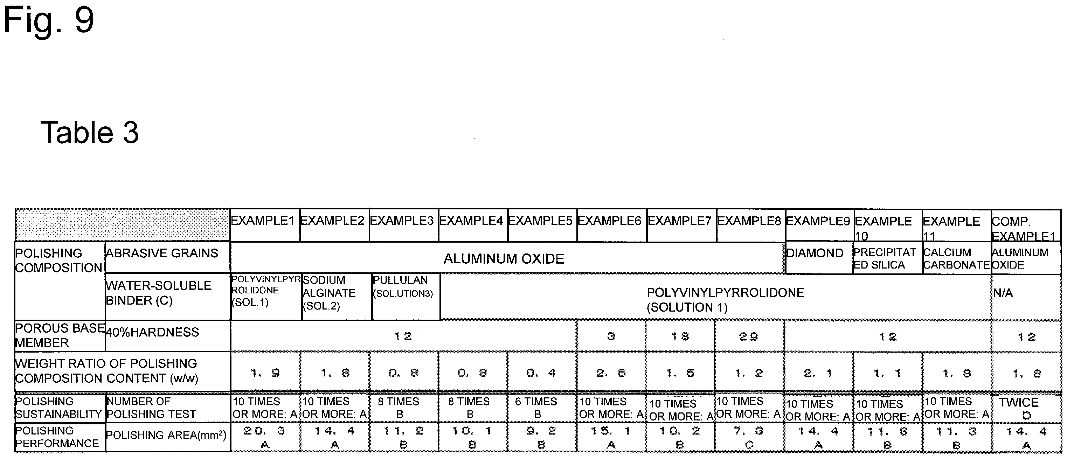

The 40% hardness of the porous base members (A), the abrasive grains (B), the water-soluble binders (C), and solutions used in Examples 1 to 11 and Comparative Example 1 are listed in Table 3 in Fig. 9.

### <Evaluation of polishing sustainability>

Disk-shaped test pieces having a diameter of 40 mm and a thickness of 3 mm were prepared by curing a base resin for dentures (Matsukaze ARBAN Co., Ltd.) using a conventional method, and were wetted dropwise with ion-exchanged water. The dental polishing instruments were then subjected to polishing tests, and the surface roughness of the polished surface was measured. The conditions of the polishing tests were set to a polishing time of 30 seconds and a rotation speed of 5000 rotations/minute.

The polishing tests were iterated, and the number of iterations repeated to achieve the surface roughness of 0.25 µm or less on the polished surface was then recorded. The number of polishing test iterations for which the test pieces succeeded in achieving the surface roughness was used as a criterion for the evaluation of polishing sustainability.

A surface roughness greater than 0.25 µm was considered as a polishing failure. In other words, when the dental polishing instruments fails to keep releasing the polishing composition, such shortage of the polishing composition becomes apparent as an increased surface roughness, because such a shortage results in a deterioration in the polishing performance.

As the surface roughness, the arithmetic mean height Sa of the contour curved surface, as stipulated in JIS B 0681-2: 2018 (corresponding to ISO 25178-2:2012), was used. The polishing sustainability was evaluated as the following four grades, the results of which are listed in Table 3 in Fig. 9. With respect to the resultant polishing sustainability, those ranked in A are determined to exhibit excellent performance; those ranked in B are determined to exhibit sufficient performance; those ranked in C are determined to be not very effective but exhibit acceptable performance; and those ranked in D are determined to exhibit unacceptable performance.
A: 10 times or more
B: 6 to 9 times
C: 3 to 5 times
D: twice or less

### <Evaluation of Polishing Performance>

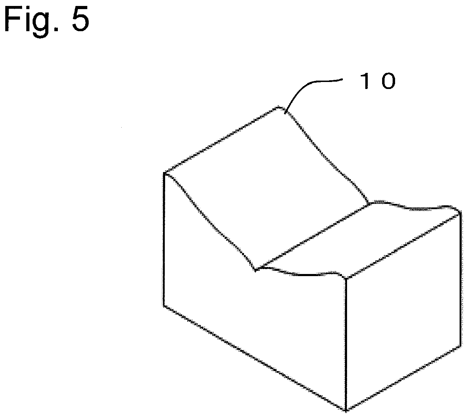

Fig. 5 is a schematic perspective view illustrating a test piece the top surface of which delineates a curved slope with a groove, as a model of the occlusal surface of a first mandibular molar. The test piece was fabricated by curing a composite resin for dental filling (Beauty fill flow plus X, Matsukaze Co., Ltd.) using a conventional method.

The entire top surface of the obtained test piece was pre-polished with water-resistant abrasive paper #1000, and then stained with an oil-based ink. After dropping ion-exchanged water to wet the groove on the top surface of the test piece, a polishing test was performed by reciprocating the dental polishing instrument in contact with the groove on the top surface. As the conditions of the polishing test, a polishing time of 60 seconds and a rotation speed of 5,000 rotations/minute were used. As an index of whether the dental polishing instrument was in contact with the curved slope of the test piece illustrated Fig. 5, which is a model of the occlusal surface of a molar, and whether a large area was polished effectively, the area polished by the dental polishing instrument was evaluated.

As the polished area, the area where the oil-based ink was removed by being brought into contact with the dental polishing instrument was calculated from image data acquired by an optical microscope, and the polished area was evaluated based on the following four-grade criteria, the results of which are indicated in Table 3 in Fig. 9. With respect to the resultant polished area, those ranked in A are determined to exhibit excellent performance; those ranked in B are determined to exhibit sufficient performance; those ranked in C are determined not to be very effective but to exhibit acceptable performance; and those ranked in D are determined to exhibit unacceptable performance.
A: 12.0 mm² or larger
B: 9.0 mm² or larger but smaller than 12.0 mm²
C: 6.0 mm² or larger but smaller than 9.0 mm²
D: Smaller than 6.0 mm²

### <Evaluation results of Examples and Comparative Examples>

Examples 1 to 7 and 9 to 11 are examples that meet all of the stipulations in the appended claims, and achieved the evaluation result either A or B, in the evaluations of the polishing sustainability as well as in the polishing performance. In other words, these examples have succeeded in polishing complicated shapes such as the occlusal surface of a molar, remarkably effectively and sustainably. Example 8 having a 40% hardness of the porous base member (A) set to 29 N, which was hard, achieved a polishing performance of C. Comparative Example 1 including no water-soluble binder achieved D, which was unacceptable, for the evaluation of the polishing sustainability.

With the dental polishing instrument according to the present invention, it is possible to polish a dental restoration or a tooth surface effectively and sustainably, without the need to use an abrasive agent such as a dental polishing paste, together with the dental polishing instrument.

### EXPLANATIONS OF LETTERS OR NUMERALS

1, 1a, 1b dental polishing instrument
2 porous base member
3 abrasive grains
4 water-soluble binder
5 test piece
6 direction of the rotational axis
7 shaft
8 shaft attachment
9 shaft
10 test piece
16 load cell

## Claims

1. A dental polishing instrument comprising:
a porous base member (A); and
a polishing composition that contains abrasive grains (B) and a water-soluble binder (C), and that is held by the porous base member.

2. The dental polishing instrument according to claim 1, wherein the abrasive grains are released from the porous base member when the polishing composition is brought into contact with water or a liquid containing alcohol.

3. The dental polishing instrument according to claim 1 or claim 2, wherein the water-soluble binder (C) is at least one selected from the group consisting of polyvinylalcohol, polyvinylpyrrolidone, polyacrylate, polyethylene glycol, alginate, pullulan, carrageenan, hydroxyethyl cellulose, and methyl cellulose.

4. The dental polishing instrument according to any one of claims 1 to 3, wherein a weight ratio of the polishing composition is set to 0.4 or higher, representing a dry weight of the porous base member (A) alone as 1.

5. The dental polishing instrument according to any one of claims 1 to 4, wherein 40% hardness of the porous base member (A), resultant of applying a compressive load to the porous base member (A) alone in accordance with JIS K 6400-2 hardness A method, is within a range of 3 N to 18 N.

6. The dental polishing instrument according to any one of claims 1 to 5, wherein the porous base member has any one of a bullet-like shape, a spindle shape, a cylindrical shape, a conical shape, a paraboloid shape, and a cup shape.

7. A polishing method comprising:
bringing the dental polishing instrument according to claim 1 into contact with a dental restoration or a tooth surface; and
polishing the dental restoration or the tooth surface while supplying water or a liquid containing alcohol to the dental abrasive.
